# EUROPÄISCHE PATENTSCHRIFT

(11) **EP 4 338 774 B1**
(45) Veröffentlichungstag und Bekanntmachung des Hinweises auf die Patenterteilung: **24.06.2026**
(21) Anmeldenummer: 22196192.3
(22) Anmeldetag: 16.09.2022
(51) Int. Cl.: A61M 15/00, A61M 11/00

(54) **AUSTRAGKOPF FÜR EINEN FLÜSSIGKEITSSPENDER SOWIE FLÜSSIGKEITSSPENDER MIT EINEM SOLCHEN AUSTRAGKOPF**
DISCHARGE HEAD FOR A LIQUID DISPENSER AND LIQUID DISPENSER HAVING SUCH A DISCHARGE HEAD
TÊTE DE DISTRIBUTION POUR UN DISTRIBUTEUR DE LIQUIDE ET DISTRIBUTEUR DE LIQUIDE DOTÉ D'UNE TELLE TÊTE DE DISTRIBUTION

(43) Veröffentlichungstag der Anmeldung: 20.03.2024
(73) Patentinhaber: Aptar Radolfzell GmbH, 78315 Radolfzell (DE)
(72) Erfinder: Greiner-Perth, Jürgen, 78244 Gottmadingen (DE)
(74) Vertreter: Witte, Weller und Partner Patentanwälte mbB Stuttgart

(56) Entgegenhaltungen:
- EP-A1- 3 978 389
- WO-A1-2009/068877
- US-B1- 6 708 846

## Beschreibung

### ANWENDUNGSGEBIET UND STAND DER TECHNIK

Die Erfindung betrifft einen Austragkopf für einen Flüssigkeitsspender sowie einen Flüssigkeitsspender mit einem Flüssigkeitsspeicher und einem Austragkopf erfindungsgemäßer Art.

Der Austragkopf eines gattungsgemäßen sowie eines erfindungsgemäßen Flüssigkeitsspenders ist zur Ankopplung oder Anbringung an einen Flüssigkeitsspeicher ausgebildet. Er verfügt über eine Düseneinheit mit einer Mehrzahl von feinen Düsenöffnungen mit einem lichten Querschnitt von jeweils höchstens 0,02 mm², durch die hindurch Flüssigkeit aus dem Flüssigkeitsspeicher in eine umgebende Atmosphäre abgegeben werden kann.

Ein solcher Austragkopf ist dafür ausgebildet, die Flüssigkeit durch die sehr kleinen Düsenöffnungen beim Austrag in feine Tröpfchen zu zerreißen, so dass ein Sprühnebel gebildet wird. Insbesondere kann eine solche Form des Austrags für Flüssigkeiten verwendet werden, die in das Auge des Benutzers, in die Nase oder in den Mund appliziert werden. Die Verwendung einer Vielzahl von Düsenöffnungen führt zu besonders feinem Nebel. Insbesondere wird hierdurch eine mittlere Tröpfchengröße erreicht, die durch eine Wirbelkammer im Bereich der Austragöffnung des Austragkopfes nicht erzielbar wäre. Die geringe Tröpfchengröße ist beispielsweise von Vorteil, um Flüssigkeit in Form eines Flüssigkeitsnebels in den Bereich der Bronchien oder der Lunge des Nutzers transportieren zu können.

Es hat sich allerdings gezeigt, dass die Verwendung von Düsenöffnungen mit dem Problem einhergehen kann, dass verbleibende Flüssigkeit nach der Verwendung in den Düsenöffnungen trocknet und Rückstände zurückbleiben, die später den weiteren Flüssigkeitsaustrag durch die Düsenöffnungen hindurch behindern, so dass die Erzeugung der gewünschten feinen Tröpfchen bei späteren Verwendungen hierdurch verschlechtert sein kann. Je nach Art der Rückstände kann der Prozess auch irreversibel sein. Der Austrag weiterer Flüssigkeit führt dann nicht zu einer Auflösung der Rückstände, sondern beeinträchtigt die Tröpfchenerzeugung nachhaltig. Neben solchen Ablagerungen kann je nach Flüssigkeit undje nach die Düsenöffnungen umgebendem Material auch Korrosion entstehen, die ebenfalls der bestimmungsgemäßen Nebelerzeugung bei nachfolgenden Austragvorgängen entgegensteht.

### AUFGABE UND LÖSUNG

Aufgabe der Erfindung ist es, einen Austragkopf und einen Flüssigkeitsspender mit einem solchen Austragkopf zur Verfügung zu stellen, bei dem die Gefahr einer solchen Verschlechterung des Austrags gemindert ist.

Gemäß der Erfindung werden ein Austragkopf und ein Flüssigkeitsspendervorgeschlagen. Diese verfügen in gattungsgemäßer Weise über eine Mehrzahl von feinen Düsenöffnungen mit einem lichten Querschnitt von jeweils höchstens 0,02 mm², durch die hindurch Flüssigkeit aus einem Flüssigkeitsspeicher in eine umgebende Atmosphäre abgegeben werden kann. Die Düsenöffnungen können parallel ausgerichtet sein. Vorzugsweise ist jedoch vorgesehen, dass die Düsenöffnungen divergierend ausgebildet sind, um hierdurch einen sich aufweitenden Sprühkonus zu bilden.

Die Düseneinheit weist vorzugsweise eine Düsenplatte auf, die von den Düsenöffnungen durchdrungen ist. Es kann sich beispielsweise um eine metallische Düsenplatte handeln, die durch galvanische Abschneidung hergestellt wurde, aber auch andere Materialien und Herstellungsverfahren sind möglich. Die Düsenplatte kann eben sein. Zur Erzeugung eines sich aufweitenden Sprühkonus kann aber auch eine gewölbte Formgebung vorgesehen sein, insbesondere mit leicht divergierender Ausrichtung der Düsenöffnungen.

Die Düseneinheit weist vorzugsweise mindestens 10 Düsenöffnungen auf, insbesondere vorzugsweise mindestens 25 Düsenöffnungen. Die Düsenöffnungen sind vorzugsweise in einer zweidimensionalen Struktur angeordnet, beispielsweise in einer Matrixform oder in Anordnung auf konzentrischen Kreisen. Vorzugsweise umfasst die Düseneinheit neben einer Düsenplatte einen separat hergestellten Träger, insbesondere in Art einer Hülse. Der Träger kann insbesondere als Kunststoffträger gestaltet sein. In den Träger ist die Düsenplatte eingesetzt. Der Träger erleichtert die Handhabung während der Montage und die Festlegung an einem tragenden Gehäuseteil des Austragkopfes.

Der Düseneinheit ist vorzugsweise mindestens ein Filter vorgeschaltet, um Partikel aus der Flüssigkeit zu beseitigen, so dass diese die Düsenöffnungen nicht verstopfen können. Findet ein Träger zum Tragen der Düsenplatte Verwendung, so trägt dieser vorzugsweise den mindestens einen Filter. Der Filter kann als Membranfilter oder Tiefenfilter ausgestaltet sein. Vorzugsweise ist einer Kaskade mehrere Filter vorgesehen, die jeweils über unterschiedliche Trenngrenzen verfügen.

Die Düseneinheit bildet das Ende des Flüssigkeitspfades, entlang dessen die Flüssigkeit aus dem Flüssigkeitsspeicher des entsprechenden Spenders in die Umgebung gelangt. Vorzugsweise ist der Düseneinheit ein Auslassventil vorgeschaltet, welches gleichzeitig das Auslassventil einer Pumpeinrichtung des Austragkopfes sein kann. Dieses Auslassventil öffnet bei Überdruck und schließt, wenn dieser Überdruck entfällt. Eine Rücksaugung von Flüssigkeit, die bereits in den Bereich der Düseneinheit gelangt ist, findet nach Ende der Betätigung bei einer solchen Gestaltung somit nicht statt.

Wie eingangs schon genannt, weist ein Austragkopf der beschriebenen Art die Problematik auf, dass im Bereich der Düsenöffnungen verbleibende Flüssigkeit bei Nichtbenutzungen zu Ablagerungen oder Korrosion in bzw. an den Düsenöffnungen führen kann, die das Sprühbild und damit auch die Wirkung des Flüssigkeitsaustrags bei späterer Verwendung beeinträchtigen.

Um dem entgegenzuwirken, ist erfindungsgemäß vorgesehen, dass der Austragkopf über eine abnehmbare und wiederaufsetzbare Schutzkappe verfügt, die im aufgesetzten Zustand die Düseneinheit gegenüber einer umgebenden Atmosphäre isoliert und die derart ausgestaltet ist, dass sie die Gefahr von Ablagerungen und Korrosion verringert.

Gemäß einer möglichen Ausgestaltung ist vorgesehen, dass die Schutzkappe einen sich außenseitig an die Düseneinheit anschließenden Isolationsraum definiert, der ein nur geringes Innenvolumen aufweist. Dieses beträgt maximal 5 ml, vorzugsweise jedoch weniger, insbesondere maximal 3 ml.

Die Wirkung dieses Isolationsraums liegt darin, dass der Verdunstung von Flüssigkeit entgegengewirkt wird. Die Flüssigkeit verbleibt somit zum überwiegenden Teil innerhalb der Düsenöffnungen und die beim Trocknen gebildeten Ablagerungen werden somit verhindert oder verringert. Je kleiner der Isolationsraum ist, der von der Kappe gebildet wird, in desto geringerem Maße findet Verdunstung statt.

Wie bereits erläutert, ist der Düseneinheit vorzugsweise ein Auslassventil vorgeschaltet. Vorzugsweise beträgt auch das Volumen im Flüssigkeitspfad zwischen dem Auslassventil und den Düsenöffnungen maximal 5 ml, insbesondere maximal 2 ml, so dass auch hier kaum Raum verbleibt, der eine Verdunstung der Flüssigkeit aus den Düsenöffnungen verhindern könnte oder in den Flüssigkeit gegebenenfalls langsam zurücklaufen könnte.

Alternativ oder vorzugsweise zusätzlich ist vorgesehen, dass die Schutzkappe eine Anlagefläche auf, die von einem ausgangsseitigen Ende der Düsenöffnungen um maximal 1 mm beabstandet ist, insbesondere vorzugsweise jedoch im aufgesetzten Zustand der Schutzkappe unmittelbar an der Düseneinheit anliegt.

Diese Anlagefläche verhindert oder vermindert zumindest die Neigung der Flüssigkeit, in den Kappeninnenraum zu verdunsten. Auch hierdurch wird somit begünstigt, dass die Flüssigkeit in den Düsenöffnungen verbleibt und somit keine schädlichen Ablagerungen hier entstehen.

Zur Bildung eines Isolationsraums mit geringem Innenvolumen kann insbesondere vorgesehen sein, dass die Schutzkappe mit einer innenseitigen Dichtfläche umlaufend an einer Außenfläche des Applikators anliegt, an dem die Düseneinheit vorgesehen ist. Insbesondere kann die Schutzkappe hierzu an ihrer Innenseite einen umlaufenden Steg aufweisen, der gegenüber der übrigen Kappeninnenfläche erhaben ist und vorzugsweise die Form einer kreiszylindrischen oder leicht konischen Ringwandung aufweist. Diese Schutzkappe legt sich im Zuge des Aufsteckens oder Aufschraubens der Kappe an einer vorzugsweise ebenfalls leicht konischen Außenfläche des Applikators an und dichtet hier ab. Es kann dabei von Vorteil sein, wenn an der inneren Wandung ein separates Dichtelement vorgesehen ist, beispielsweise in Form eines elastischen Dichtrings oder eines anderweitigen Körpers aus einem TPE-Material. Hierdurch ist eine besonders gute Abdichtung zu erzielen.

Die genannte innere Wandung kann, gegebenenfalls ohne das genannte Dichtelement, einstückig Teil eines einheitlichen Kappenkörpers sein, der auch die übrigen Wandungen der Kappe und Kopplungsflächen zur Ankopplung an ein Gehäuse des Spenders bildet. Denkbar ist aber auch, dass die Schutzkappe ein inneres Kappenelement aufweist, welches den Isolationsraum bildet und welches sich demzufolge insbesondere an den Außenflächen des Applikators anlegen kann, um den Isolationsraum zu schaffen. Dieses innere Kappenelement ist nicht einstückig mit einem äußeren Kappenelement verbunden.

Das innere Kappenelement und das äußere Kappenelementsind vorzugsweise fest miteinander verbunden. Denkbar sind allerdings auch Gestaltungen, bei denen die beiden Kappenelemente beweglich miteinander verbunden sind. Insbesondere kann das innere Kappenelement drehbar am äußeren Kappenelement angebracht sein. Dies gestattet es beispielsweise, eine Schraubkappe vorzusehen, deren äußeres Kappenelement mit einem Gewinde versehen ist, wobei das die Abdichtung des Isolationsraums bewirkende innere Kappenelement beim Aufschrauben der Kappe nach Kontakt mit dem Applikator zu diesem rotativ ortsfest verbleiben kann. Möglich ist auch eine Gestaltung, bei der das innere Kappenelement und das äußere Kappenelement vollständig getrennt sind und separat gehandhabt werden können.

Wie eingangs erläutert wurde, erlaubt es der Isolationsraum, das Volumen stark zu begrenzen, welches eine Verdunstung der Flüssigkeit aus den Düsenöffnungen zulassen könnte, so dass die Flüssigkeit in den Düsenöffnungen verbleibt. Dies ist insbesondere dann sehr hilfreich, wenn die Flüssigkeit zwar im Falle von Verdunstung schädliche Ablagerung zurückließe, ihr Verbleib in den Düsenöffnungen aber keine Korrosion zur Folge hätte.

Sofern die Kombination der Flüssigkeit des Flüssigkeitsspenders und des Materials der Düseneinheit, insbesondere der Düsenplatte, die Gefahr von Korrosion mit sich bringt, kann es jedoch vorteilhaft sein, die Flüssigkeit aus den Düsenöffnungen zu entfernen.

Daher wird auch eine Gestaltung der Schutzkappe vorgeschlagen, bei der die Schutzkappe derart an die Formgebung des Applikators angepasst ist, dass der von der Schutzkappe und dem Applikator begrenzte Isolationsraum beim Aufsetzen in einer Zwischenstellung durch umlaufende Anlage der Schutzkappe am Applikator geschlossen wird und dann eine Verkleinerung erfährt. Dies ist beispielsweise zu erreichen, indem der Isolationsraum in einer Endstellung bei vollständig aufgesetzter Schutzkappe ein Innenvolumen aufweist, welches weniger als 70% der Größe des Innenvolumens des Isolationsraums in der genannten Zwischenstellung beträgt, vorzugweise weniger als 60% oder gar weniger als 50%.

Durch eine solche Verkleinerung des Isolationsraums wird die darin befindliche Luft beim Aufsetzen der Schutzkappe komprimiert. Der Druck der Flüssigkeit in den Düsenöffnungen steigt daraufhin, so dass beim Übergang aus der Zwischenstellung in die Endstellung die Luft im Isolationsraum in die Düsenöffnungen eindringt und die Flüssigkeit von dort verdrängt. Sofern ein den Düsenöffnungen vorgeschaltetes Auslassventil vorgesehen ist, stellt es einen Vorteil dar, wenn dieses durch den mittels der Schutzkappe erzielbaren außenseitigen Überdruck geöffnet werden kann und die Flüssigkeit hierdurch in den Flüssigkeitsspeicher zurückgedrückt werden kann.

Eine weitere Möglichkeit zur schnellen Entfernung der Flüssigkeit aus den Düsenöffnungen liegt in einer Gestaltung, bei der innerhalb des Isolationsraums ein feuchtigkeitsabsorbierendes Element angeordnet ist. Hierbei kann es sich insbesondere um einen schwammartigen oder anderweitig porösen Körper handeln, insbesondere mit hydrophilen Eigenschaften. Besonders bevorzugt werden Elemente aus Zellulose.

Das feuchtigkeitsabsorbierende Element kann dem Zweck dienen, die Feuchtigkeit in der Luft zu reduzieren und somit ein schnelles Verdunsten zu ermöglichen. Hierfür muss das feuchtigkeitsabsorbierende Element nicht zwingend in sehr engem Kontakt mit der Düseneinheit stehen. Von Vorteil ist allerdings eine Gestaltung, bei der das feuchtigkeitsabsorbierende Element unmittelbar an den Düsenöffnungen anliegt und die Flüssigkeit gleichsam aus den Düsenöffnungen saugt. Hierdurch werden Ablagerungen vermieden.

Die Verwendung eines feuchtigkeitsabsorbierenden Elements ist daher insbesondere dann von Vorteil, wenn die Flüssigkeit nicht primär durch Ablagerungen die Austragcharakteristik der Düsenöffnungen verschlechtert, sondern aufgrund von Korrosion.

Die Erfindung betrifft neben einer Gestaltung mit einer Kappe mit einem Isolationsraum geringen Volumens und/oder einer den Düsenöffnungen nahen Anlagefläche auch eine Gestaltung mit einem potenziell größeren Kappeninnenraum, wobei aber auch hier in oben beschriebener Weise ein feuchtigkeitsabsorbierendes Element vorgesehen ist.

Das beschriebene feuchtigkeitsabsorbierende Element muss nicht in unmittelbarer Nähe der Düseneinheit vorgesehen sein. Von Vorteil ist es jedoch, wenn das Element nahe an der Düseneinheit angeordnet ist oder sogar an dieser anliegt. Es hat sich gezeigt, dass ein Abstand von weniger als 0,5 mm, insbesondere ein Abstand von 0,2 mm oder gar ein Abstand von weniger als 0,1 mm eine besonders schnelle Entfernung der Flüssigkeit aus den Düsenöffnungen ermöglicht. Bei geringem Abstand verdunstet die Flüssigkeit nicht, sondern wird aus den Düsenöffnungen und insbesondere von einer Außenseite der Düsenplatte entfernt. Dementsprechend ist auch die Gefahr von Ablagerungen hierdurch reduziert.

Es ist möglich, das feuchtigkeitsabsorbierende Element über einen Verbindungskanal, der die Kappenwandung durchdringt, mit einer umgebenden Atmosphäre zu verbinden. Hierdurch kann die aufgenommene Feuchtigkeit an die Umgebung abgegeben werden. Ein solcher Verbindungskanal durchbricht vorzugsweise eine Stirnseite der Schutzkappe und wird insbesondere vorzugsweise vom feuchtigkeitsabsorbierenden Element überdeckt.

Ein erfindungsgemäßer Austragkopf kann insbesondere derart ausgestaltet sein, dass er eine Basis aufweist, gegenüber der eine Betätigungseinheit entlang einer Hauptachse zum Zwecke der Betätigung niederdrückbar ist, wobei die Düsenöffnungen im Mittel in Richtung der Hauptachse ausgerichtet sind. Insbesondere sind die Düsenöffnungen vorzugsweise an einem distalen Ende eines Applikators wie eines Nasalapplikators vorgesehen, der Teil der Betätigungseinheit ist.

Die Schutzkappe ist in einem solchen Fall vorzugweise zum Aufsetzen oder Aufschrauben in Richtung der Hauptachse vorgesehen, so dass hierdurch der oben genannte Isolationsraum geschlossen bzw. das flüssigkeitsabsorbierende Element an die Düsenöffnungen angenähert wird und vorzugsweise bei vollständig aufgesetzter Kappe an diesen anliegt.

Eine andere Bauweise eines Austragkopfes sieht vor, dass der Austragkopf eine Basis aufweist, gegenüber der eine Betätigungseinheit ebenfalls entlang einer Hauptachse zum Zwecke des Austrags niederdrückbar ist, wobei in diesem Falle die Düsenöffnungen gegenüber der Hauptachse angewinkelt sind. Vorzugsweise schließt eine mittlere Ausrichtung der Düsenöffnungen mit der Hauptachse einen Winkel zwischen 70° und 110° ein. Bei einer solchen Gestaltung ist somit bezogen auf die Hauptachse und Haupterstreckungsrichtung des Flüssigkeitsspenders ein in etwa seitlicher Austrag der Flüssigkeit vorgesehen.

In einem solchen Falle ist ebenfalls eine Schutzkappe möglich, die in Richtung der Hauptachse auf das Gehäuse des Austragkopfes aufsetzbar ist. Da es hierdurch jedoch erschwert ist, ein flüssigkeitsabsorbierendes Element oder die Wandung eines Isolationsraums in der aufgesetzten Stellung an der richtigen Position relativ zu den Düsenöffnungen zu platzieren, wird es bei einer angewinkelten Konfiguration von Hauptachse und mittlerer Erstreckungsrichtung der Düsenöffnungen bevorzugt, wenn die Schutzkappe zur Anbringung in einer gegenüber der Hauptachse angewinkelten Fügerichtung vorgesehen ist, vorzugsweise in Richtung der mittleren Erstreckungsrichtung der Düsenöffnung. In einem solchen Falle wird die Kappe also gleichsam seitlich auf den Austragkopf aufgesetzt.

Ein erfindungsgemäßer Austragkopf ist vorzugweise dafür vorgesehen, mit einem Flüssigkeitsspeicher gekoppelt zu werden, insbesondere indem die Basis des Austragkopfes durch eine Schnapp- oder Gewindeverbindung auf einen Flüssigkeitsspeicher aufgesetzt wird. Auch eine einstückige Verbindung der Basis und des Flüssigkeitsspeichers ist möglich.

Der Austragkopf weist insbesondere vorzugsweise eine Betätigungseinheit auf, die gegenüber der Basis verlagerbar ist, insbesondere in Richtung der Basis niederdrückbar. Diese Betätigungsbewegung führt zur Förderung von Flüssigkeit aus dem Flüssigkeitsspeicher zur Düseneinheit und somit zum Flüssigkeitsaustrag. Dabei besteht eine Möglichkeit darin, dass die Flüssigkeit mittels einer Pumpeinrichtung mit einer volumetrisch veränderlichen Pumpkammer, die eingangsseitig und ausgangsseitig mit jeweils einem Ventil versehen ist, gefördert wird. Eine andere Möglichkeit besteht darin, dass die Flüssigkeit im Flüssigkeitsspeicher unter Druck gelagert ist und der Austragkopf über ein Auslassventil verfügt, welches durch die Betätigungseinheit geöffnet werden kann, so dass die druckbeaufschlagte Flüssigkeit zur Düseneinheit gedrückt wird und dort abgegeben wird.

Die Betätigung der Betätigungseinheit erfolgt vorzugsweise über eine manuell zu betätigende Betätigungsfläche, die an einem distalen Ende der Betätigungseinheit vorgesehen sein kann. Auch eine Gestaltung mit einer seitlichen und gegebenenfalls umlaufenden Fingerauflage ist möglich.

Ein Flüssigkeitsspeicher, der mit einem Austragkopf der beschriebenen Art ausgerüstet ist, weist einen Flüssigkeitsspeicher auf, vorzugsweise mit einem Innenvolumen von weniger als 200 ml, insbesondere mit einem Innenvolumen von weniger als 100 ml. Er ist im befüllten Zustand vorzugsweise mit einer kosmetischen oder pharmazeutischen Flüssigkeit befüllt.

Das Patentdokument WO2009/068877A1 wird hiermit zur Kenntnis genommen.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1A bis 1C zeigen ein erstes Ausführungsbeispiel eines erfindungsgemäßen Spenders.
Fig. 2A bis 2C zeigen zu einem weiteren Ausführungsbeispiel den Ablauf des Aufsetzens einer Schutzkappe.
Fig. 3A bis 3B zeigen ein weiteres Ausführungsbeispiel und Fig. 3C eine Variante hierzu.
Fig. 4A bis 4C zeigen ein weiteres Ausführungsbeispiel.
Fig. 5A bis 5C zeigen ein weiteres Ausführungsbeispiel sowie eine besondere Variante hierzu in Fig. 5C.
Fig. 6A bis 7B zeigen zwei weiteren erfindungsgemäße Spender.
Fig. 8A bis 8C zeigen ein letztes Ausführungsbeispiel der Erfindung.

### DETAILLIERTE BESCHREIBUNG DER AUSFÜHRUNGSBEISPIELE

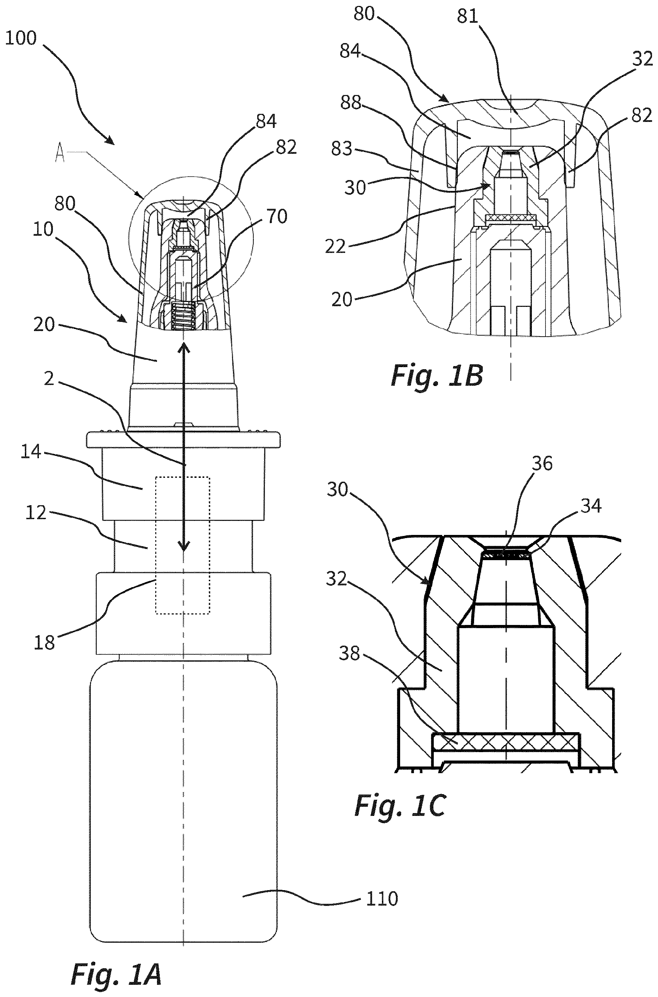

Fig. 1A bis 1C zeigen eine erste Variante eines erfindungsgemäßen Flüssigkeitsspenders 100 mit einem Flüssigkeitsspeicher 110 und einem hieran angebrachten Austragkopf 10.

Der Austragkopf 10 verfügt über eine Basis 12, an der der Flüssigkeitsspeicher 110 befestigt ist, und über eine gegenüber dieser Basis 12 in Richtung des Pfeils 2 gegen eine Federkraft verlagerbare Betätigungseinheit 14 mit einer Fingerauflagefläche 16 und einem Applikator 20, vorliegend einem langgestreckten, schlanken Nasalapplikator 20.

Durch Niederdrücken der Betätigungseinheit 14 wird eine nicht näher dargestellte Pumpeinrichtung 18 betätigt, die Flüssigkeit aus dem Flüssigkeitsspeicher 110 zu einer Düseneinheit 30 am distalen Ende des Nasalapplikators 20 fördert. Wenn der Flüssigkeitsspeicher 110 als Druckspeicher ausgebildet ist, könnte alternativ zur Pumpeinrichtung auch eine Ventileinrichtung 18 vorgesehen sein, die durch Niederdrücken der Betätigungseinheit 14 geöffnet wird und dann die druckbeaufschlagte gelagerte Flüssigkeit zur Düseneinheit 30 strömen lässt.

Der Düseneinheit 30 vorgeschaltet, ist im Falle der Gestaltung der Fig. 1A bis 1C ein Auslassventil mit einem federbeaufschlagten Ventilkörper 70. Dieses Auslassventil öffnet, wenn ein Innenseitiger Flüssigkeitsdruck einen Öffnungsdruck erreicht und der Ventilkörper 70 infolgedessen gegen die Kraft der Druckfeder nach unten verlagert wird.

Der Flüssigkeitsspender 100 weist eine Schutzkappe 80 auf, die auf die Betätigungseinheit 14 aufgesetzt ist und im vorliegenden Ausführungsbeispiel als Steckkappe ausgebildet ist.

Die Düseneinheit 30 des Flüssigkeitsspenders 100 ist in Fig. 1C in vergrößerter Darstellung gezeigt. Die Düseneinheit 30 verfügt über einen hülsenförmigen Kunststoffträger 32, der am oberen Ende des Applikators 20 innerhalb einer stirnseitigen Durchbrechung befestigt ist. Der Kunststoffträger 32 umgibt einen Zufuhrkanal, an dessen innenseitigem Ende ein Filter 38 eingesetzt ist und an dessen außenseitigem Ende eine Düsenplatte 34vorgesehen ist. Die Düsenplatte ist von einer Vielzahl feiner Düsenöffnungen 36 durchdrungen. Die Düseneinheiten 30 der im Folgenden beschriebenen weiteren Ausführungsbeispiele weist einen entsprechenden Aufbau auf.

Wie anhand der Fig. 1B ersichtlich ist, verfügt die Schutzkappe über eine Stirnfläche 81 und eine äußere Mantelfläche 83. Zusätzlich ist innenseitig an der Stirnfläche 81 ein umlaufender Steg 82 vorgesehen, der im aufgesetzten Zustand der Schutzkappe 80 mit einem distalen Ende des Nasalapplikators 20 in umlaufenden Kontakt gelangt und hierdurch gemeinsam mit der Stirnfläche des Applikators 20 und der dort angeordneten Düseneinheit 30 einen Isolationsraum 84 definiert. Das Innenvolumen dieses Isolationsraums liegt bei etwa 2 ml.

Wenn der Flüssigkeitsspender verwendet worden ist, so verbleibt nach der Nutzung ein Teil der Flüssigkeit innerhalb der Düsenöffnungen 36. Je nach Art der Flüssigkeit und Material der Düsenplatte 34 kann dies unkritisch sein. Ist die Flüssigkeit, die mittels des Flüssigkeitsspenders 100 ausgetragen wird, jedoch dergestalt, dass bei ihrer Verdunstung Ablagerungen zurückbleiben, beispielsweise auskristallisierte Salze, so kann dies das Sprühbild bei nachfolgenden Anwendungen negativ beeinflussen.

Der sehr kleine Isolationsraum 84, der durch die Schutzkappe 80 definiert wird, verhindert wirksam, dass die Flüssigkeit in den Düsenöffnungen 36 nach Gebrauch verdunstet, sofern die Schutzkappe 80 aufgesetzt ist. Die Flüssigkeit verbleibt somit bei Nichtgebrauch zumindest für einige Stunden oder Tage in den Düsenöffnungen 36, ohne dass es hier zu Ablagerungen kommt.

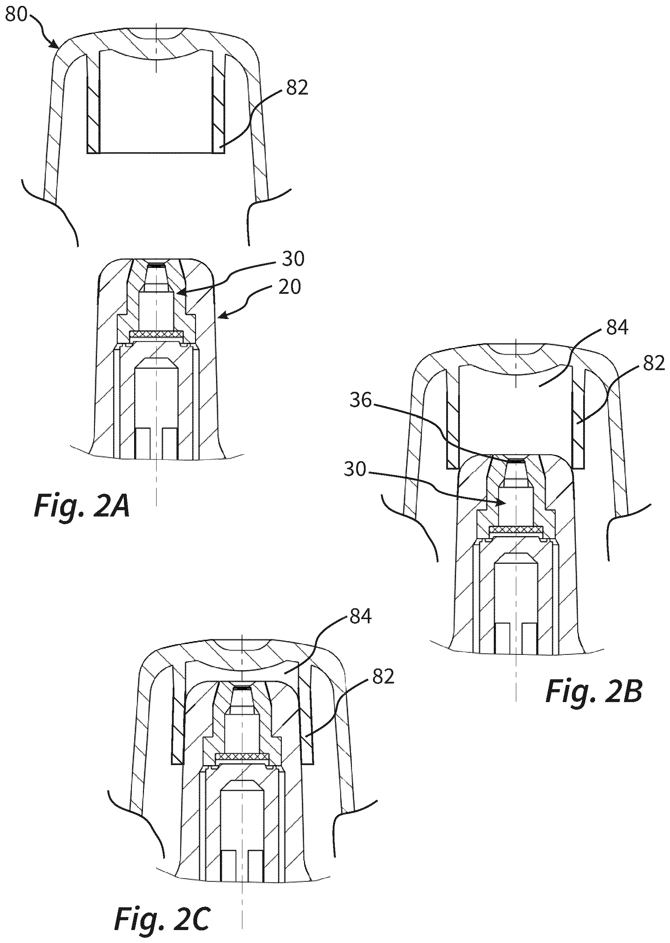

Eine äußerlich ähnliche Bauweise ist in den Fig. 2A bis 2C verdeutlicht. Trotz der Ähnlichkeit zur Gestaltung der Fig. 1A bis 1C ist die Funktionsweise hier eine andere.

Die Schutzkappe 80 ist im Falle der Gestaltung der Fig. 2A bis 2C mit einem in Aufsteckrichtung besonders langen Steg 82 versehen. Dies bewirkt, dass der Innenraum 84, der vom umlaufenden Steg 82 begrenzt wird, beim Aufstecken der Schutzkappe 80 bereits im Zwischenzustand der Fig. 2B gegenüber dem übrigen Kappeninnenraum isoliert wird. Beim fortgesetzten Aufstecken der Schutzkappe 80 bis in den abschließenden Zustand der Fig. 2C wird das Innenvolumen des Innenraums 84 deutlich reduziert, vorliegend um etwa 80% bis 90%. Dementsprechend wird die hier vorhandene Luft komprimiert und der Luftdruck erhöht sich, so dass diese Luft von außen in die Düsenöffnungen 36 der Düseneinheit 30 hineingedrückt wird und die dort zuvor verbliebene Flüssigkeit nach innen verdrängt. Somit verringert sich die Gefahr, dass es in einer sich an die Nutzung anschließenden Lagerphase hier zu Korrosion kommt.

Die Feder des Ventilkörpers 70d des Spenders 100 gemäß Fig. 2A bis 2C ist vorzugsweise derart ausgelegt, dass der im Innenraum 84 bewirkte Druckanstieg ausreicht, um das Ventil zu öffnen, so dass die Flüssigkeit zurück in den Spender strömen kann.

Die Gestaltung des Flüssigkeitsspenders der Fig. 2A und 2B unterscheidet sich hinsichtlich der Schutzkappe 80 von der Gestaltung der Fig. 1A bis 2C. Bei dieser Gestaltung ist kein Isolationsraum vorgesehen. Stattdessen ist an der Innenseite der Schutzkappe 80 ein flüssigkeitsabsorbierendes Element 86, insbesondere ein Zellulosekörper vorgesehen. Dieser Zellulosekörper wird durch einen Tragring 82 getragen und ist derart angeordnet, dass er bei aufgesetzter Kappe in geringem Abstand zur Düsenplatte 34 und den Düsenöffnungen 36 angeordnet ist. Auf einer von der Düsenplatte 34 wegweisenden Seite ist der Tragring 82 durch einen Verbindungskanal 90 unterbrochen, so dass durch das flüssigkeitsabsorbierende Element 86, aufgenommene Flüssigkeit nach außen in den umgebenden Kappeninnenraum abgegeben werden kann.

Bei dieser Gestaltung liegt der Zweck der Kappengestaltung nicht darin, den Verbleib von Flüssigkeit in den Düsenöffnungen zu gewährleisten. Vielmehr begünstigt die Kappe ein schnelles Trocknen, indem verdunstete Flüssigkeit im flüssigkeitsabsorbierenden Element 86 aufgenommen wird und hierdurch ein Anstieg der Luftfeuchtigkeitzwischen dem flüssigkeitsabsorbierenden Element 86 und der Düsenplatte 34 verhindert wird. Um das Entfernen der Flüssigkeit noch weiter zu beschleunigen, kann das flüssigkeitsabsorbierende Element 86 spaltfrei an der Düsenplatte 34 anliegen.

Die Verwendung einer solche Gestaltung, die ein schnelles Entfernen von Flüssigkeit aus den Düsenöffnungen begünstigt, ist ebenso wie die Gestaltung der Fig. 2A bis 2C insbesondere dann zweckmäßig, wenn die Flüssigkeit korrosive Wirkung auf die Düsenplatte 34 entfaltet. Dies kann beispielsweise im Falle von metallischen Düsenplatten der Fall sein. In einem solchen Falle sind nicht Ablagerungen von Flüssigkeitsinhalten an den Düsenöffnungen 36 das Hauptproblem, sondern die Korrosion durch in den Düsenöffnungen 36 verbleibende Flüssigkeit.

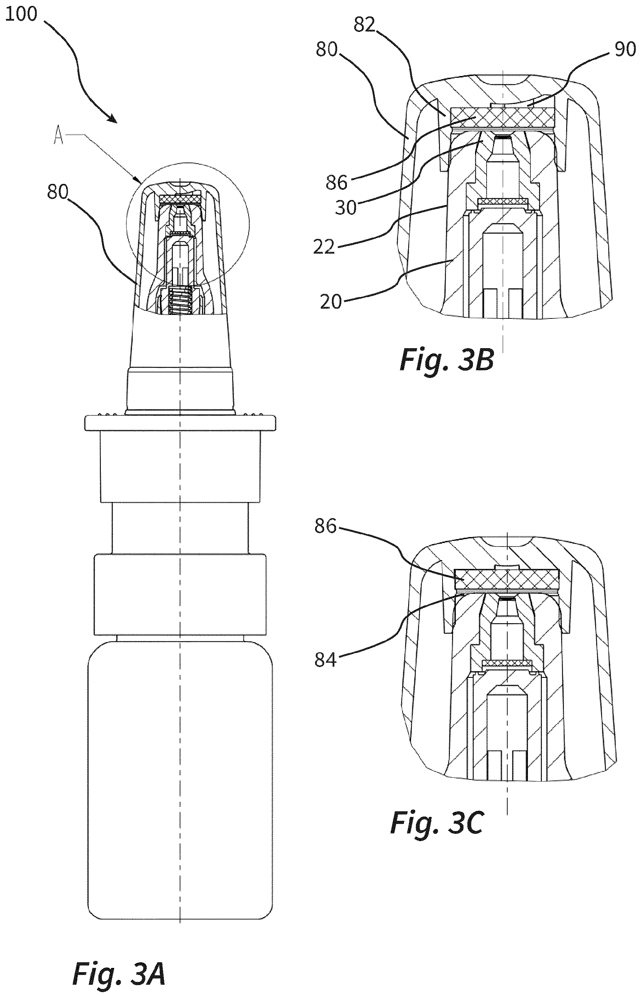

Bei der Gestaltung der Fig. 3A und 3B ist der genannte Verbindungskanal 90 vorgesehen, mittels dessen Flüssigkeit aus dem flüssigkeitsabsorbierenden Element 86 in den übrigen Kappeninnenraum abgeführt werden kann. Fig. 3C zeigt eine Alternative, bei der dieser Verbindungskanal 90 nicht vorgesehen ist. Die Gestaltung weist daher einen geschlossenen Isolationsraum 84 entsprechend der Gestaltung der Fig. 1A bis 1C auf, die demgegenüber lediglich um das flüssigkeitsabsorbierende Element 86 ergänzt ist.

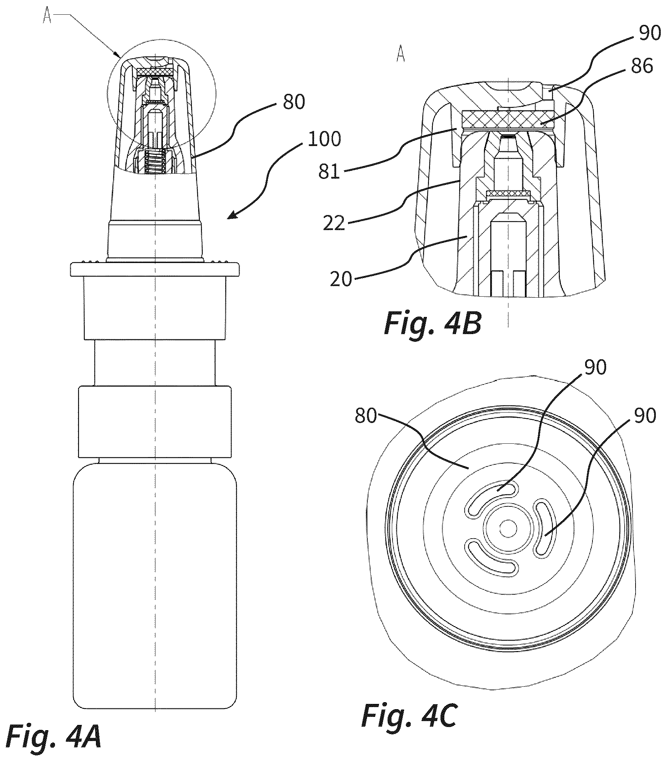

Bei der Gestaltung der Fig. 4A bis 4C ist wiederum vorgesehen, dass ein schnelles Abtrocknen der Flüssigkeit durch ein flüssigkeitsabsorbierendes Element 86 begünstigt wird. Im Unterschied zu der vorangegangenen Gestaltung ist hier allerdings vorgesehen, dass der Verbindungskanal 90 das Flüssigkeitsabsorbierende Element 86 nicht mit einem anderen Teil des Kappeninnenraums verbindet, sondern mit einer Umgebung. Hierfür sind in der in Fig. 4C ersichtlichen Weise insgesamt drei Öffnungen in der Stirnfläche der Kappe vorgesehen, die Verbindungskanäle 90 bilden.

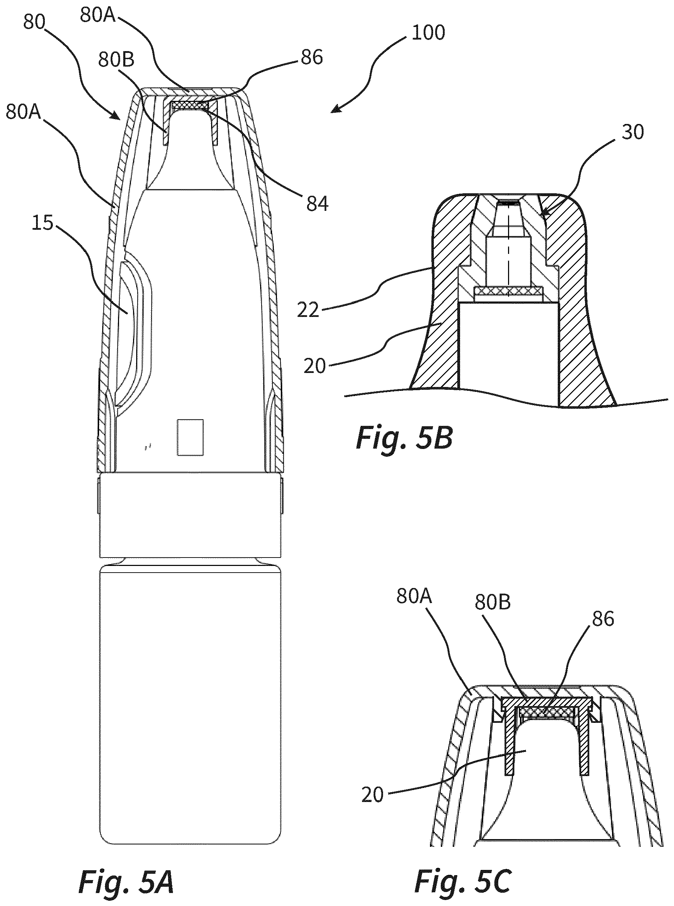

Fig. 5A bis 5C zeigen ein weiteres Ausführungsbeispiel und eine abgewandelte Variante hiervon. Bei dieser Gestaltung ist die Austragvorrichtung selbst etwas anders aufgebaut, da sie als Side-Actuation-Austragvorrichtung mit einer seitlichen Betätigungstaste 15 gestaltet ist. Ein Niederdrücken einer Betätigungseinheit, an der der Applikator vorgesehen ist, ist hier also nicht gegeben.

Übereinstimmend mit den vorangegangenen Ausführungsbeispiel weist der Flüssigkeitsspender 100 der Fig. 4 einen Nasalapplikator 20 auf, in dem eine Düseneinheit 30 entsprechend jener der Fig. 1A bis 1C vorgesehen ist, wie anhand der geschnittenen Darstellung der Fig. 5B zu erkennen ist. Wie auch bei der Gestaltung der Fig. 1A bis 1C ist vorgesehen, dass bei aufgesetzter Schutzkappe 80 ein Isolationsraum 84 im Bereich der Düseneinheit 30 ein geringes Volumen definiert. Die Besonderheit hier liegt allerdings darin, dass dieser Isolationsraum 84 durch ein inneres Kappenelement 80B gemeinsam mit dem Applikator gebildet wird, wobei dieses innere Kappenelement 80B getrennt von einem äußeren Kappenelement 80A gebildet ist, welches das Hauptelement der Schutzkappe 80 darstellt und bei aufgesetzter Schutzkappe 80 mit dem Gehäuse der Austragvorrichtung mechanisch gekoppelt ist.

Das innere Kappenelement 80B kann fest mit dem äußeren Kappenelement 80A verbunden sein, so dass sie als ein gemeinsam gehandhabtes und zueinander dauerhaft ortsfestes Element funktionieren.

Das innere Kappenelement 80B kann alternativ auch als vollständig separates Kappenelement 80A ausgebildet sein. Das Abnehmen der Schutzkappe 80 durch Abnehmen ihres Hauptbestandteils 80A bewirkt in einem solchen Falle noch nicht das Abnehmen des inneren Kappenelements 80B. Dieses muss nachträglich separat abgenommen werden.

Eine dritte Möglichkeit ist in Fig. 5C verdeutlicht. Hier ist das innere Kappenelement 80B über eine Rastverbindung mit dem äußeren Kappenelement 80A verbunden, so dass diese stets gemeinsam gehandhabt werden. Allerdings verbleibt das innere Kappenelement 80B drehbar gegenüber dem äußeren Kappenelement 80A. Dies kann insbesondere vorteilhaft sein, wenn die Schutzkappe 80 bzw. deren äußeres Kappenelement mittels eines Gewindes an der Austragvorrichtung befestigt wird und demzufolge beim Aufsetzen der Schutzkappe 80 gedreht wird. Hier gestattet es die verbleibende Drehbeweglichkeit des inneren Kappenelements 80B gegenüber dem äußeren Kappenelement 80A, dass nach Kontakt des inneren Kappenelements 80B mit dem Applikator 20 die Drehbewegung des inneren Kappenelements 80B endet und beim fortgesetzten Aufschrauben des äußeren Kappenelements 80A die Kappenelemente 80A, 80B demzufolge gegeneinander drehen.

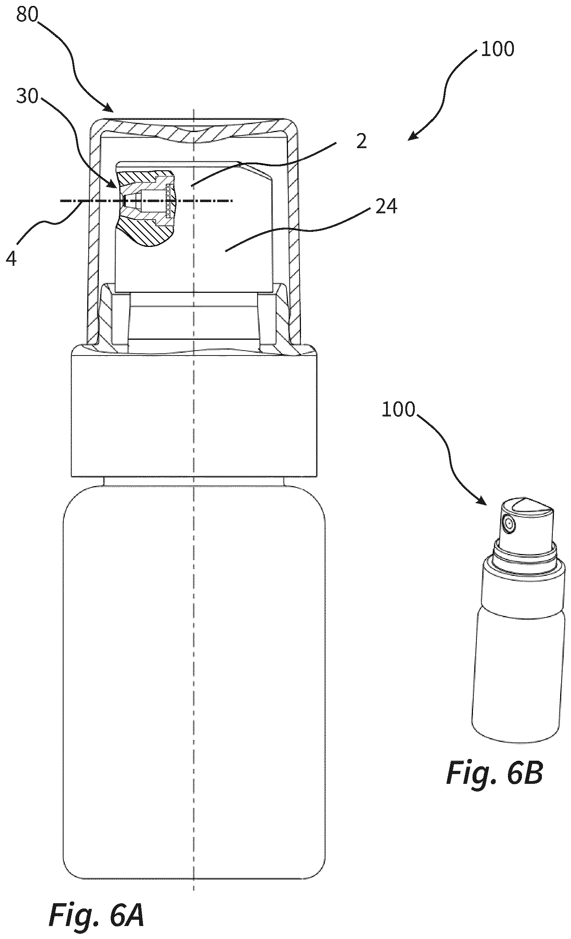

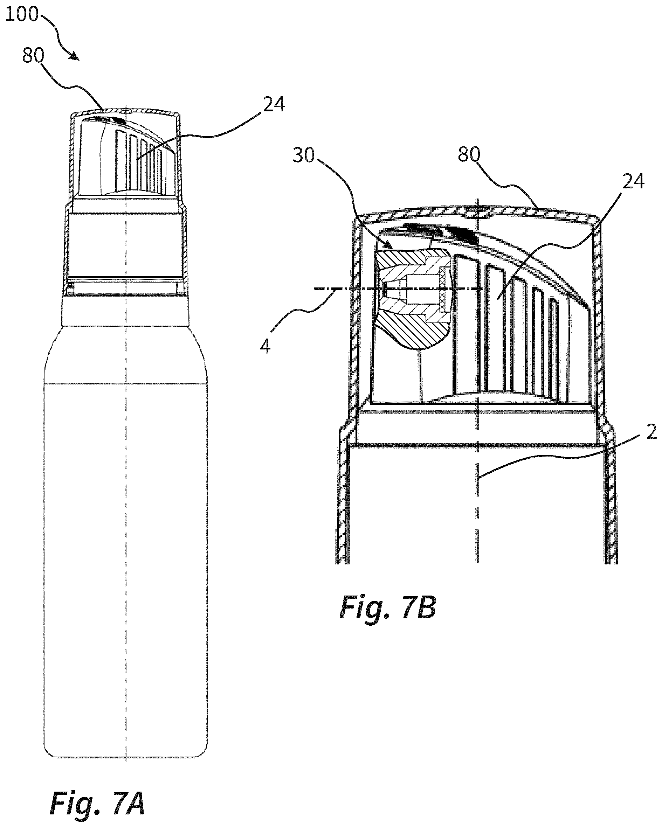

Die Fig. 6A, 6B und 7A und 7B zeigen weitere Flüssigkeitsspender 100, die über Düseneinheiten 30 verfügen, durch die hindurch Flüssigkeit in eine Umgebung abgegeben werden kann, wobei abweichend von den vorangegangenen Gestaltungen eine Betätigungsrichtung 2 hier mit einer mittleren Austragrichtung 4, die durch die Erstreckungsrichtung der Düsenöffnungen 36 definiert wird, einen Winkel von ca. 90° einschließt. Die Düseneinheit 30 ist an einem niederdrückbaren Betätigungsdrücker 24 vorgesehen, mittels dessen eine Pumpeinrichtung des Spenders betätigt wird.

Auch bei dieser Gestaltung ist eine Schutzkappe 80 vorgesehen, die im aufgesetzten Zustand luftdicht gegenüber eine Umgebung abschließt und somit die Gefahr verringert, dass die gesamte Flüssigkeit aus den Düsenöffnungen 36 der Düseneinheit 30 verdunstet und nachteilige Rückstände verbleiben.

Die grundsätzliche Bauweise bei der Gestaltung der Fig. 7A und 7B ähnelt der der Fig. 6A und 6B. Hier ist jedoch besonderer Wert darauf gelegt worden, dass die Schutzkappe 80 den Betätigungsdrücker 24 mit der Düseneinheit 30 möglichst eng umgibt, so dass das Volumen des Kappeninnenraums im aufgesetzten Zustand gering ist. Hierdurch wird gewährleistet, dass nur eine sehr geringe Menge der in den Düsenöffnungen 36 verbleibenden Flüssigkeit verdunsten kann, bis die hierdurch bewirkte Luftfeuchtigkeit im Kappeninnenraum einer weiteren Verdunstung entgegensteht.

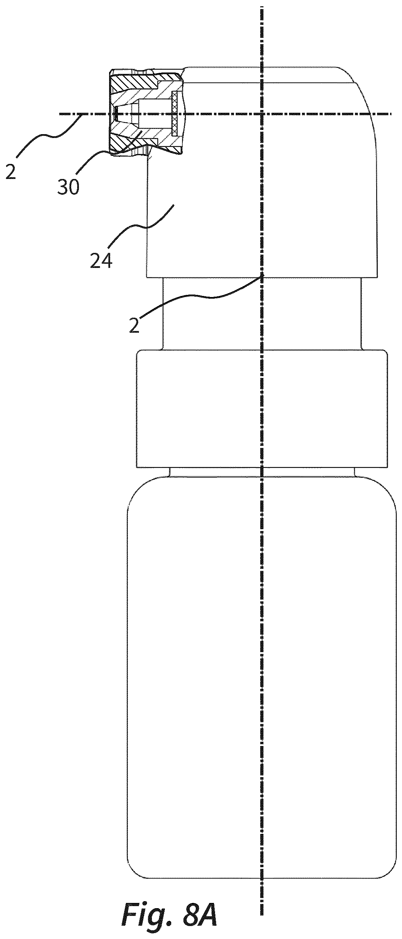

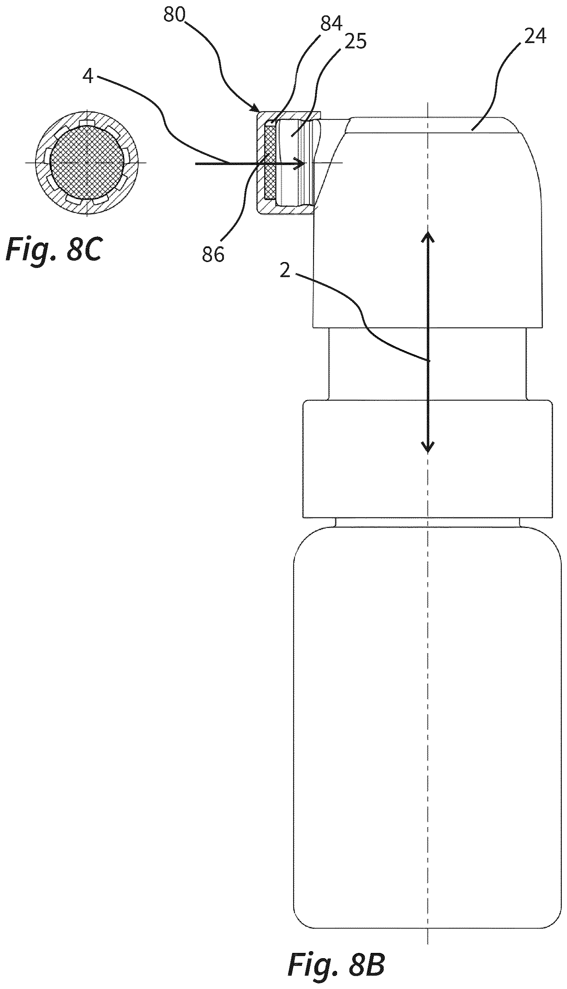

Fig. 8A bis 8C zeigen eine weitere Version eines erfindungsgemäßen Flüssigkeitsspenders 100 mit einem erfindungsgemäßen Austragkopf. Ähnlich den Gestaltungen der Fig. 6A bis 7B ist auch hier ein Betätigungsdrücker 24 vorgesehen, der zum Zwecke des Flüssigkeitsaustrags niedergedrückt wird. Die Düseneinheit 30 ist an diesem Betätigungsdrücker vorgesehen und weist eine mittlere Austragrichtung 2 auf, die zur Betätigungsrichtung in einem Winkel von ca. 90° steht.

Wie Fig. 7B zeigt, ist die Schutzkappe 80 hier abweichend von den Fig. 6A bis 7B so gestaltet, dass sie in Richtung der mittleren Austragrichtung 2, also von der Seite, aufgesetzt wird. Der Betätigungsdrücker weist einen seitlich hervorstehenden Fortsatz 25 auf, innerhalb dessen die Düseneinheit 30 angeordnet ist. Die Außenseite dieses Fortsatzes 25 ist mit einer umlaufenden Vertiefung vorgesehen, so dass die Schutzkappe 80 hier einschnappen kann.

Die Schutzkappe 80 definiert im aufgesetzten Zustand mit dem Applikatorfortsatz 25 einen Isolationsraum 84 mit sehr geringem Innenvolumen. Innerhalb dieses Isolationsraums 84 ist ein feuchtigkeitsabsorbierendes Element 86 angeordnet, das den Isolationsraum zu einem großen Teil ausfüllt und insbesondere im aufgesetzten Zustand der Fig. 7B unmittelbar an diese Düseneinheit 30 angrenzt. Das feuchtigkeitsabsorbierende Element 86 ist daher in der Lage, nach dem Aufsetzen Flüssigkeit von der Düseneinheit 30 unmittelbar aufzusaugen und insbesondere auch aus den Düsenöffnungen 36 der Düseneinheit 30 herauszuziehen.

## Patentansprüche

1. Austragkopf (10) für einen Flüssigkeitsspender (100) mit den folgenden Merkmalen:
a. der Austragkopf (10) ist zur Ankopplung an einen Flüssigkeitsspeicher (110) ausgebildet, und
b. der Austragkopf (10) verfügt über eine Düseneinheit (30) mit einer Mehrzahl von feinen Düsenöffnungen (36) mit einem lichten Querschnitt von höchstens 0,02 mm², durch die hindurch Flüssigkeit aus dem Flüssigkeitsspeicher (110) in eine umgebende Atmosphäre abgegeben werden kann, und
c. der Austragkopf (10) verfügt über eine abnehmbare und wiederaufsetzbare Schutzkappe (80), die im aufgesetzten Zustand die Düseneinheit (30) gegenüber einer umgebenden Atmosphäre isoliert,
**gekennzeichnet durch** eines der folgenden weiteren Merkmale:
d. die Schutzkappe (80) definiert einen Isolationsraum (84), der sich im aufgesetzten Zustand der Schutzkappe (80) an die Düseneinheit (30) anschließt und der ein Innenvolumen max. 5 ml aufweist, oder
e. die Schutzkappe (80) weist eine Anlagefläche auf, die im aufgesetzten Zustand der Schutzkappe (80) an der Düseneinheit (30) anliegt oder von einem ausgangsseitigen Ende der Düsenöffnungen (36) um maximal 1 mm beabstandet ist.

2. Austragkopf (10) nach Anspruch 1 mit den folgenden weiteren Merkmalen:
a. die Düseneinheit (30) ist an einem distalen Ende eines Applikators (20) vorgesehen, insbesondere am distalen Ende eines Nasalapplikators (20), und
b. die Schutzkappe (80) liegt mit einer innenseitigen Dichtfläche (88) an einer Außenfläche (22) des Applikators (20) an,
vorzugweise mit dem folgenden zusätzlichen Merkmal:
c. die Schutzkappe (80) weist ein separates Dichtelement auf, welches die innenseitige Dichtfläche (88) bildet.

3. Austragkopf (10) nach Anspruch 1 oder 2 mit den folgenden weiteren Merkmalen:
a. die Schutzkappe (80) weist ein inneres Kappenelement (80B) auf, welches den Isolationsraum (84) bildet, und
b. die Schutzkappe (80) weist ein äußeres Kappenelement (80A) auf, innerhalb dessen das innere Kappenelement (80B) angeordnet ist,
vorzugsweise mit dem folgenden zusätzlichen Merkmal:
c. das innere Kappenelement (80B) und das äußere Kappenelement (80A) sind fest miteinander verbunden.

4. Austragkopf (10) nach einem der Ansprüche 2 oder 3 mit dem folgenden weiteren Merkmal:
a. die Schutzkappe (80) weist an einer Innenseite einen umlaufenden Steg (82) auf, der im aufgesetzten Zustand umlaufend am Applikator (20) des Austragkopfes (10) zur Anlage kommt und den Isolationsraum (84) außenseitig begrenzt.

5. Austragkopf (10) nach einem der Ansprüche 2 bis 4 mit den folgenden weiteren Merkmalen:
a. die Schutzkappe (80) ist derart an die Formgebung des Applikators (20) des Austragkopfes (10) angepasst, dass der von der Schutzkappe (80) und dem Applikator (20) begrenzte Isolationsraum (84) beim Aufsetzen in einer Zwischenstellung durch umlaufende Anlage der Schutzkappe am Applikator (20) geschlossen wird, und
b. der Isolationsraum (84) weist in einer Endstellung bei vollständig aufgesetzter Schutzkappe (80) ein Innenvolumen auf, welches weniger als 70% der Größe des Innenvolumens des Isolationsraums (84) in der Zwischenstellung beträgt, vorzugweise weniger als 60%, so dass beim Übergang aus der Zwischenstellung in die Endstellung Luft im Isolationsraum (84) komprimiert wird und durch die Düsenöffnungen (36) in den Austragkopf (10) hineingedrückt wird.

6. Austragkopf (10) nach einem der vorstehenden Ansprüche mit dem folgenden weiteren Merkmal:
a. innerhalb des Isolationsraums (84) ist ein feuchtigkeitsabsorbierendes Element (86) angeordnet.

7. Austragkopf (10) für einen Flüssigkeitsspender (100) nach dem Oberbegriff des Anspruchs 1, **gekennzeichnet durch** das folgende weitere Merkmal:
a. die Schutzkappe (80) verfügt über ein feuchtigkeitsabsorbierendes Element (86).

8. Austragkopf (10) nach Anspruch 6 oder 7 mit dem folgenden weiteren Merkmal:
a. das feuchtigkeitsabsorbierende Element (86) und die Düseneinheit (30) sind durch einen Zwischenraum getrennt, dessen Innenvolumen höchstens 0,1 ml beträgt.

9. Austragkopf (10) nach einem der Ansprüche 6 bis 8 mit dem folgenden weiteren Merkmal:
a. das feuchtigkeitsabsorbierende Element (86) liegt unmittelbar an einer Außenseite der Düseneinheit (30) im Bereich der Düsenöffnungen (36) an.

10. Austragkopf (10) nach einem der Ansprüche 7 bis 9 mit dem folgenden weiteren Merkmal:
a. auf einer der Düseneinheit (30) abgewandten Seite des feuchtigkeitsabsorbierenden Elements (86) ist mindestens ein Verbindungskanal (90) vorgesehen, der eine Verbindung zu einem Kappeninnenraum oder zu einer umgebenden Atmosphäre schafft.

11. Austragkopf (10) nach einem der Ansprüche 6 bis 10 mit dem folgenden weiteren Merkmal:
a. das feuchtigkeitsabsorbierende Element (86) besteht aus einem porösen Material und/oder aus Zellulose.

12. Austragkopf (10) nach einem der vorstehenden Ansprüche mit den folgenden weiteren Merkmalen:
a. der Austragkopf (10) weist eine Basis (12) auf, gegenüber der eine Betätigungseinheit (14) entlang einer Hauptachse (2) niederdrückbar ist, und
b. die Düsenöffnungen (36) sind in Richtung der Hauptachse (2) ausgerichtet.

13. Austragkopf (10) nach einem der Ansprüche 11 bis 11 mit den folgenden weiteren Merkmalen:
a. der Austragkopf (10) weist eine Basis (12) auf, gegenüber der eine Betätigungseinheit (14) entlang einer Hauptachse (2) niederdrückbar ist, und
b. die Düsenöffnungen (36) sind gegenüber der Hauptachse (2) angewinkelt, insbesondere in einem Winkel zwischen 70° und 110°,
vorzugsweise mit dem folgenden zusätzlichen Merkmal:
c. die Schutzkappe (80) ist zur Anbringung in einer gegenüber der Hauptachse (2) angewinkelten Fügerichtung (4) vorgesehen.

14. Austragkopf (10) nach einem der vorstehenden Ansprüche mit mindestens einem der folgenden zusätzlichen Merkmale:
a. die Düseneinheit (30) weist eine Düsenplatte (34) auf, die von den Düsenöffnungen (36) durchdrungen ist, und/oder
b. die Düseneinheit (30) weist mindestens 25 Düsenöffnungen (36) auf, und/oder
c. die Düseneinheit (30) weist einen Kunststoffträger (32) auf, in den die Düsenplatte (34) eingesetzt ist, und/oder
d. die Düseneinheit (30) weist mindestens einen Filter (38) stromaufwärts der Düsenöffnungen (36) auf.
e. der Düseneinheit (30) ist ein Auslassventil (70) vorgeschaltet.

15. Flüssigkeitsspender (100) zum Austrag einer Flüssigkeit in zerstäubter Form, insbesondere zum Austrag einer pharmazeutischen oder kosmetischen Flüssigkeit, mit den folgenden Merkmalen:
a. der Flüssigkeitsspender (100) weist einen Flüssigkeitsspeicher (110) auf, und
b. der Flüssigkeitsspender (100) weist einen Austragkopf (10) auf,
**gekennzeichnet durch** das folgende Merkmal:
c. der Austragkopf ist nach einem der Ansprüche 1 bis 14 ausgebildet,
vorzugsweise mit mindestens einem der folgenden zusätzlichen Merkmale:
d. der Flüssigkeitsspeicher weist ein Volumen von 200 ml oder weniger auf, und/oder
e. der Flüssigkeitsspeicher ist mit einer pharmazeutischen oder einer kosmetischen Flüssigkeit befüllt, und/oder
f. der Austragkopf (10) ist über eine Schnappverbindung oder eine Schraubverbindung mit dem Flüssigkeitsspeicher (110) oder der Austragkopf (10) weist eine Basis auf, die einstückig mit dem Flüssigkeitsspeicher (110) verbunden ist.

## Claims

1. Delivery head (10) for a fluid dispenser (100) with the following features:
a. the delivery head (10) is configured for coupling to a fluid store (110), and
b. the delivery head (10) has a nozzle unit (30) with a plurality of fine nozzle openings (36), each with a clear cross-section of maximum 0.02 mm², through which fluid can be discharged from the fluid store (110) to a surrounding atmosphere, and
c. the delivery head (10) has a removable and refittable protective cap (80) which, when fitted, isolates the nozzle unit (30) from a surrounding atmosphere,
**characterized by** one of the following further features:
d. the protective cap (80) defines an isolation space (84) which, when the protective cap (80) is fitted, adjoins the nozzle unit (30) and has an inner volume of maximum 5 ml, or
e. the protective cap (80) has a contact face which, when the protective cap (80) is fitted, lies against the nozzle unit (30) or is spaced from an output-side end of the nozzle openings (36) by maximum 1 mm.

2. Delivery head (10) according to Claim 1, with the following further features:
a. the nozzle unit (30) is provided at a distal end of an applicator (20), in particular at the distal end of a nasal applicator (20), and
b. the protective cap (80) lies with an inner sealing surface (88) against an outer face (22) of the applicator (20),
preferably with the following additional feature:
c. the protective cap (80) has a separate sealing element which forms the inner sealing surface (88).

3. Delivery head (10) according to Claim 1 or 2, with the following further features:
a. the protective cap (80) has an inner cap element (80B) which forms the isolation space (84), and
b. the protective cap (80) has an outer cap element (80A) inside which the inner cap element (80B) is arranged,
preferably with the following additional feature:
c. the inner cap element (80B) and the outer cap element (80A) are fixedly connected together.

4. Delivery head (10) according to one of Claims 2 or 3, with the following further feature:
a. the protective cap (80) has on an inside a circumferential web (82) which, when fitted, comes to rest circumferentially on the applicator (20) of the delivery head (10) and delimits the isolation space (84) on the outside.

5. Delivery head (10) according to any of Claims 2 to 4, with the following further features:
a. the protective cap (80) is adapted to the shape of the applicator (20) of the delivery head (10) such that the isolation space (84) delimited by the protective cap (80) and the applicator (20) is closed during attachment in an intermediate position by a circumferential contact of the protective cap (80) on the applicator (20), and
b. in a fully fitted end position of the protective cap (80), the isolation space (84) has an inner volume which amounts to less than 70%, preferably less than 60%, of the inner volume of the isolation space (84) in the intermediate position, so that on transition from the intermediate position to the end position, air inside the isolation space (84) is compressed and pushed through the nozzle openings (36) into the delivery head (10).

6. Delivery head (10) according to any of the preceding claims, with the following further feature:
a. a moisture-absorbing element (86) is arranged inside the isolation space (84).

7. Delivery head (10) for a fluid dispenser (100) according to the preamble of Claim 1, **characterized by** the following further feature:
a. the protective cap (80) has a moisture-absorbing element (86).

8. Delivery head (10) according to Claim 6 or 7, with the following further feature:
a. the moisture-absorbing element (86) and the nozzle unit (30) are separated by intermediate space with an inner volume of maximum 0.1 ml.

9. Delivery head (10) according to any of Claims 6 to 8, with the following further feature:
a. the moisture-absorbing element (86) lies directly against an outside of the nozzle unit (30) in the region of the nozzle openings (36).

10. Delivery head (10) according to any of Claims 7 to 9, with the following further feature:
a. on a side of the moisture-absorbing element (86) facing away from the nozzle unit (30), at least one connecting channel (90) is provided which creates a connection to a cap interior or to a surrounding atmosphere.

11. Delivery head (10) according to any of Claims 6 to 10, with the following further feature:
a. the moisture-absorbing element (86) consists of a porous material and/or cellulose.

12. Delivery head (10) according to any of the preceding claims, with the following further features:
a. the delivery head (10) has a base (12) against which an actuating unit (14) can be pressed down along a main axis (2), and
b. the nozzle openings (36) are oriented in the direction of the main axis (2).

13. Delivery head (10) according to any of Claims 11 to 11, with the following further features:
a. the delivery head (10) has a base (12) against which an actuating unit (14) can be pressed down along a main axis (2), and
b. the nozzle openings (36) are angled relative to the main axis (2), in particular by an angle between 70° and 110°,
preferably with the following additional feature:
c. the protective cap (80) is provided for attachment in a joining direction (4) which is angled relative to the main axis (2).

14. Delivery head (10) according to any of the preceding claims, with at least one of the following additional features:
a. the nozzle unit (30) has a nozzle plate (34) through which the nozzle openings (36) are made, and/or
b. the nozzle unit (30) has at least 25 nozzle openings (36), and/or
c. the nozzle unit (30) has a plastic carrier (32) in which the nozzle plate (34) is inserted, and/or
d. the nozzle unit (30) has at least one filter (38) upstream of the nozzle openings (36),
e. an outlet valve (70) is arranged upstream of the nozzle unit (30).

15. Fluid dispenser (100) for delivering a fluid in atomized form, in particular for delivering a pharmaceutical or cosmetic fluid, with the following features:
a. the fluid dispenser (100) has a fluid store (110), and
b. the fluid dispenser (100) has a delivery head (10),
**characterized by** the following feature:
c. the delivery head is configured according to any of Claims 1 to 14,
preferably with at least one of the following additional features:
d. the fluid store has a volume of 200 ml or less, and/or
e. the fluid store is filled with a pharmaceutical or a cosmetic fluid, and/or
f. the delivery head (10) is connected to the fluid store (110) via a snap connection or screw connection, or the delivery head (10) has a base which is integrally connected to the fluid store (110).

## Revendications

1. Tête de décharge (10) pour un distributeur de liquide (100) avec les caractéristiques suivantes :
a. la tête de décharge (10) est formée pour être couplée à un réservoir de liquide (110), et
b. la tête de décharge (10) dispose d'une unité de buse (30) avec une multitude de fines ouvertures de buse (36) avec une section transversale libre de 0,02 mm² au maximum, à travers lesquelles du liquide provenant du réservoir de liquide (110) peut être fourni dans une atmosphère environnante, et
c. la tête de décharge (10) dispose d'un capuchon de protection (80) amovible et replaçable, qui, dans l'état placé, isole l'unité de buse (30) d'une atmosphère environnante,
**caractérisée par** une des caractéristiques supplémentaires suivantes :
d. le capuchon de protection (80) définit un espace d'isolation (84) qui, lorsque le capuchon de protection (80) est placé, se raccorde à l'unité de buse (30) et qui présente un volume intérieur de 5 ml au maximum, ou
e. le capuchon de protection (80) comporte une surface d'appui, qui, dans l'état placé du capuchon de protection (80), repose sur l'unité de buse (30) ou est espacée de 1 mm au maximum d'une extrémité côté sortie des ouvertures de buse (36).

2. Tête de décharge (10) selon la revendication 1, avec les caractéristiques supplémentaires suivantes :
a. l'unité de buse (30) est prévue sur une extrémité distale d'un applicateur (20), en particulier sur l'extrémité distale d'un applicateur nasal (20), et
b. le capuchon de protection (80) repose par une surface d'étanchéité côté intérieur (88) sur une surface extérieure (22) de l'applicateur (20),
de préférence avec la caractéristique supplémentaire suivante :
c. le capuchon de protection (80) comporte un élément d'étanchéité séparé qui forme la surface d'étanchéité côté intérieur (88).

3. Tête de décharge (10) selon la revendication 1 ou 2 avec les caractéristiques supplémentaires suivantes :
a. le capuchon de protection (80) comporte un élément de capuchon intérieur (80B) qui forme l'espace d'isolation (84), et
b. le capuchon de protection (80) comporte un élément de capuchon extérieur (80A) à l'intérieur duquel est disposé l'élément de capuchon intérieur (80B),
de préférence avec la caractéristique supplémentaire suivante :
c. l'élément de capuchon intérieur (80B) et l'élément de capuchon extérieur (80A) sont reliés fixement l'un à l'autre.

4. Tête de décharge (10) selon l'une des revendications 2 ou 3, avec la caractéristique supplémentaire suivante :
a. le capuchon de protection (80) comporte, sur un côté intérieur, une nervure périphérique (82) qui, dans l'état placé, vient en appui en périphérie sur l'applicateur (20) de la tête de décharge (10) et délimite côté extérieur l'espace d'isolation (84).

5. Tête de décharge (10) selon l'une des revendications 2 à 4, avec les caractéristiques supplémentaires suivantes :
a. le capuchon de protection (80) est adapté à la forme de l'applicateur (20) de la tête de distribution (10) de telle manière que l'espace d'isolation (84) délimité par le capuchon de protection (80) et l'applicateur (20) est fermé, lors de la mise en place dans une position intermédiaire, par un appui périphérique du capuchon de protection (80) sur l'applicateur (20), et
b. l'espace d'isolation (84) comporte, dans une position finale à l'état complètement monté du capuchon de protection (80), un volume intérieur qui est inférieur à 70 %, de préférence inférieur à 60 %, de la taille du volume intérieur de l'espace d'isolation (84) dans la position intermédiaire, de telle sorte que, lors du passage de la position intermédiaire dans la position finale, l'air est comprimé dans l'espace d'isolation (84) et est poussé à travers les ouvertures de buse (36) dans la tête de distribution (10).

6. Tête de décharge (10) selon l'une des revendications précédentes, avec la caractéristique supplémentaire suivante :
a. un élément absorbant l'humidité (86) est disposé à l'intérieur de l'espace d'isolation (84).

7. Tête de décharge (10) pour un distributeur de liquide (100) selon le préambule de la revendication 1, **caractérisée par** la caractéristique supplémentaire suivante :
a. le capuchon de protection (80) dispose d'un élément absorbant l'humidité (86).

8. Tête de décharge (10) selon la revendication 6 ou 7, avec la caractéristique supplémentaire suivante :
a. l'élément absorbant l'humidité (86) et l'unité de buse (30) sont séparés par un espace intermédiaire dont le volume intérieur est de 0,1 ml au maximum.

9. Tête de décharge (10) selon l'une des revendications 6 à 8, avec la caractéristique supplémentaire suivante :
a. l'élément absorbant l'humidité (86) repose directement sur un côté extérieur de l'unité de buse (30) dans la zone des ouvertures de buse (36).

10. Tête de décharge (10) selon l'une des revendications 7 à 9, avec la caractéristique supplémentaire suivante :
a. sur un côté de l'élément absorbant l'humidité (86) opposé à l'unité de buse (30), au moins un canal de liaison (90) est prévu, lequel établit une liaison avec l'espace intérieur de capuchon ou avec l'atmosphère environnante.

11. Tête de décharge (10) selon l'une des revendications 6 à 10, avec la caractéristique supplémentaire suivante :
a. l'élément absorbant l'humidité (86) est constitué d'un matériau poreux et/ou de cellulose.

12. Tête de décharge (10) selon l'une des revendications précédentes, avec les caractéristiques supplémentaires suivantes :
a. la tête de décharge (10) comporte une base (12) par rapport à laquelle une unité d'actionnement (14) peut être enfoncée le long d'un axe principal (2), et
b. les ouvertures de buse (36) sont orientées dans la direction de l'axe principal (2).

13. Tête de décharge (10) selon l'une des revendications 11 à 11, avec les caractéristiques supplémentaires suivantes :
a. la tête de décharge (10) comporte une base (12) par rapport à laquelle une unité d'actionnement (14) peut être enfoncée le long d'un axe principal (2), et
b. les orifices de buse (36) sont inclinés par rapport à l'axe principal (2), en particulier selon un angle entre 70° et 110°°,
de préférence avec la caractéristique supplémentaire suivante :
c. le capuchon de protection (80) est prévu pour être installé dans une direction d'assemblage (4) inclinée par rapport à l'axe principal (2).

14. Tête de décharge (10) selon l'une des revendications précédentes, avec au moins une des caractéristiques supplémentaires suivantes :
a. l'unité de buse (30) comporte une plaque de buse (34), qui est traversée par les ouvertures de buse (36), et/ou
b. l'unité de buse (30) comporte au moins 25 ouvertures de buse (36), et/ou
c. l'unité de buse (30) comporte un support en matière plastique (32), dans lequel est insérée la plaque de buse (34), et/ou
d. l'unité de buse (30) comporte au moins un filtre (38) en amont des ouvertures de buse (36).
e. une soupape de sortie (70) est branchée en amont de l'unité de buse (30).

15. Distributeur de liquide (100) destiné à décharger un liquide sous forme atomisée, en particulier destiné à décharger un liquide pharmaceutique ou cosmétique, avec les caractéristiques suivantes :
a. le distributeur de liquide (100) comporte un réservoir de liquide (110), et
b. le distributeur de liquide (100) comporte une tête de décharge (10),
**caractérisé par** la particularité suivante :
c. la tête de décharge est formée selon l'une des revendications 1 à 14,
de préférence avec au moins une des caractéristiques supplémentaires suivantes :
d. le réservoir de liquide comporte un volume de 200 ml ou moins, et/ou
e. le réservoir de liquide est rempli d'un liquide pharmaceutique ou cosmétique, et/ou
f. la tête de décharge (10) est reliée par une liaison à déclic ou une liaison par vissage au réservoir de liquide (110) ou la tête de décharge (10) comporte une base qui est reliée d'une seule pièce au réservoir de liquide (110).
